# EUROPEAN PATENT APPLICATION

(11) **EP 4 119 188 A1**
(43) Date of publication of application: **18.01.2023**
(21) Application number: 21894819.8
(22) Date of filing: 09.08.2021
(51) Int. Cl.: A61N 1/36, A61N 1/05

(54) **SUB-TYPE ARTIFICIAL RETINA DEVICE, AND DRIVING METHOD AND MANUFACTURING METHOD THEREFOR**

(30) Priority: 20.11.2020 KR 20200156872
(71) Applicant: CELLICO Inc., Seongnam-si, Gyeonggi-do 13449 (KR)
(72) Inventor: JANG, Lee-Woon, Seongnam-si Gyeonggi-do 13588 (KR); KANG, Ho-Sung, Incheon 21912 (KR)
(74) Representative: Schmitt-Nilson Schraud Waibel Wohlfrom Patentanwälte Partnerschaft mbB
(86) International application number: PCT/KR2021/010464
(87) International publication number: WO 2022/108042

(57) **Abstract**

Provided are a new sub-type artificial retina device, and a driving method and a manufacturing method of the sub-type artificial retina device capable of minimizing cross-talk. The sub-type artificial retina device and the driving method thereof can effectively control cross-talk, compared to conventional methods. In addition, the manufacturing method of the sub-type artificial retina device can manufacture a sub-type artificial retina device capable of effectively controlling cross-talk through a simple process, and can reduce costs.

## Description

### Technical Field

The present disclosure relates to a sub-type artificial retina device, a driving method thereof and a manufacturing method thereof.

### Background Art

The retina is the light sensitive layer of nerve tissue of the human eye. There are approximately one hundred million visual cells in the retina, and this area is about 6.25 cm². Rod cells which account for the majority of the visual cells convert images into electrical signals, and these signals are transmitted to the brain through the optic nerve at a speed of about 480 km/h. The brain interprets minute electrical signals to understand images and to gauge objects. Because the retina is one of the tissues with the most blood supply per unit area, requires lots of energy sources and wastes generated by chemical reactions in nerves should be removed. However, when the wastes are not removed clearly in the retinal vessels or choroidal vessels, it may induce various eye related diseases.

Retinitis pigmentosa (RP), which is a retinal disease, is a progressive retinal degenerative disease caused by disfunction of photoreceptors distributed in the retina and appears in both eyes, photoreceptors and the retinal pigment epithelium being the main lesions of retinitis pigmentosa. It has been reported that the prevalence of RP is one in 5,000 people worldwide. As another retinal disease, age-related macular degeneration (AMD) is one of the three major blindness diseases. Recently, the prevalence of AMD is rapidly increasing due to an aging population. Unlike low-vision patients due to RP, AMD patients often go through vision deterioration in a relatively short period of time. It has been reported that the degree of physical impairment and psychological atrophy caused by the eyes in AMD patients is greater than that of other diseases.

Recently, various treatments such as gene therapy, stem cell therapy, drug therapy, and the like have been suggested to treat blind patients. However, most blind patients have already suffered damage to the retinal visual cell layer, so the period available for gene therapy or drug treatment has passed. However, in the case of diseases such as RP and AMD, only the visual cell layer which is the outer layer of the retina is damaged. So, in a case in which the function of the visual cell layer is replaced, there is the possibility of restoring eyesight. Therefore, an artificial retina for restoring eyesight by inducing electrical stimulation of the visual cell layer of the retina in a blind patient is a promising new treatment.

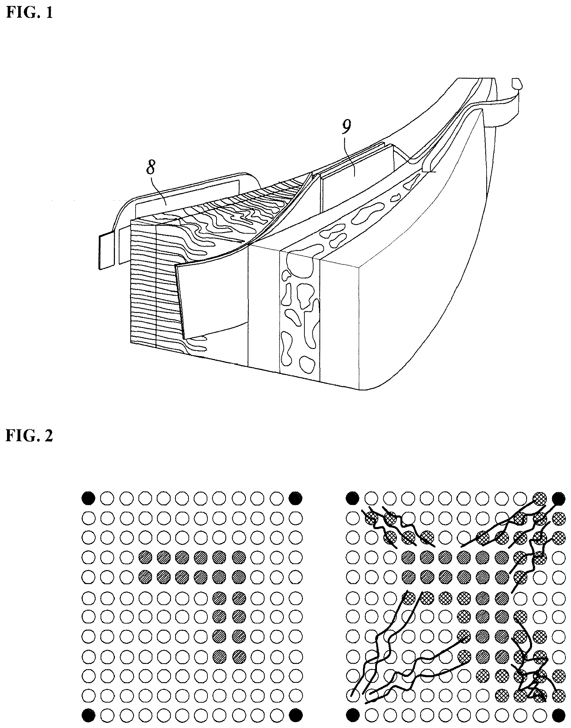

Referring to FIG. 1, the artificial retina is divided into an epi-type (epi-retinal) and a sub-type (sub-retinal) according to an installation location. The epi-type artificial retina is located in front of the retina and is numbered 8 in FIG. 1. The sub-type artificial retina is located on the visual cell layer behind the retina and is numbered 9 in FIG. 1. The epi-type artificial retina stimulates the ganglion cell layer among the retinal cells, and the sub-type artificial retina stimulates the rear bipolar cell layer. In the epi-type artificial retina, since the nerve cell stimulator is located in front of the retina, the intermediate signal processing of nerve cells in the internal retinal layer does not run. Therefore, the epi-type artificial retina is separately provided with an external camera. The external camera is mounted on glasses. Image information obtained from the camera wirelessly reaches the intraocular microelectrode array through an induction coil, and directly stimulates retinal ganglion cells without the intermediate signal processing of the nerve cells of the internal retinal layer. On the other hand, the threshold for responding to electrical stimulation is varied for each patient, and the degree of the electrical stimulation to be applied is also varied, depending on the damaged retinal cell. The epi-type artificial retina independently controls electrodes with an external image processor.

Accordingly, there is an advantage in that the electric pulse amplitude can be freely changed according to patients or damaged areas. As conventional art, Second Sight Argus II products, sold in the United States, can independently control 64 electrodes, and also control the magnitude of electrical stimulation generated by each electrode. However, since the epi-type artificial retina is very thin and fragile, it is difficult to fix the electrodes. In addition, the epi-type artificial retina may be exposed to the vitreous cavity since being located inside the retina, electrical stimulation cannot be transferred since the epi-type artificial retina is surrounded by the fibrous tissues. Moreover, in a case in which electrical stimulation is applied from the upper surface of the retina, the retinal nerve fiber layer is stimulated to spread the signal, or cells of several layers in the retina are stimulated simultaneously, so it is difficult to improve spatial resolution. Since the epi-type artificial retina cannot utilize the intraretinal signal processing process, there may be a difference between the shape of a stimulating electrode grid and the shape that a patient feels actually. Thus, it may be necessary to provide customized image processing for each patient. Therefore, the epi-type artificial retina requires more parts than those of the sub-type artificial retina and a signal transmitting unit to connect the various parts.

In the sub-type artificial retina, as shown in FIG. 1, the photodiode array is located on the photoperceptive cell layer below the retinal cell layer. The sub-type artificial retina is designed to simply replace the function of photoreceptors and targets bipolar cells for primary electrical stimulation. Thus, the sub-type artificial retina is designed to integrate a photodiode detecting light and a stimulation electrode so that current flowing from the photodiode directly flows to the electrode to stimulate the retinal nerve cells. The photodiode array performs a function similar to a CMOS image sensor. The size of dark current generated by each photodiode cell is varied according to intensity of light, and the current is changed into a biphasic current pulse serving as an action potential while passing through a conversion circuit. The sub-type artificial retina recognizes an object by using the existing visual transmission path through information processing of the bipolar cells and the internal retinal layer, thereby allowing a user to feel natural. In addition, the microelectrode array is inserted into the eyeball to enable natural eye movement. Thus, the sub-type artificial retina has advantages in that a user has to turn not only the eyes but the entire head in the direction of the object in order to see and recognize the object in a case of a system having a small camera mounted on glasses and in that the sub-type artificial retina is relatively physiological and natural. Furthermore, the sub-type artificial retina can realize high resolution since the number of pixels made by a subretinal stimulation method is the largest in comparison with artificial retinas made so far.

As conventional art, the Alpha IMS model successfully commercialized by Retina Implant in Germany has 1500 photodiode arrays and biphasic current generating arrays matching with the photodiode arrays. However, according to a clinical test, it was reported that actual resolution was lower than resolution of a 63-channel epi-type artificial retina. In a case in which the epi-type artificial retina stimulates, a picture captured by the camera is converted into a digital signal through image processing, and then, is converted into a serial digital signal through encoding to be transmitted to an artificial retina. A decoder in the artificial retina analyzes the packet of the digital signal coming from the outside and sequentially sends command signals to each stimulator. In this instance, the stimulator receiving a command generates biphasic current, and output terminals of the other stimulators waiting for the command are shorted with the return electrode to prevent the residual charges from spreading widely.

Now, the photodiode array in the sub-type artificial retina will be described. Light enters the photodiode array simultaneously, and the photodiode array generates biphasic currents simultaneously. Meanwhile, in the sub-type artificial retina, the return electrode serving as a ground is distant from a distal end of a chip or an electrode array. In general, the return electrodes are located to serve as a ground at edges of the rectangular chip. In such a situation, in a case in which the plurality of stimulation electrodes are stimulated in a certain region simultaneously, currents flow into the return electrodes, and retinal bipolar cells which should not be stimulated are stimulated, thereby generating the cross-talk phenomenon to create image blur. FIG. 2 illustrates a photodiode array illustrating the cross-talk phenomenon.

In FIG. 2, it is assumed that the shape that the user wants to recognize is '┐.'That is, the '┐'-shaped image reaches the sub-type artificial retina after passing the intermediate cell layer of the retina, and stimulations are simultaneously input to the photodiode in which a plurality of pixels, for instance, 1000 pixels, are arranged. The stimulation electrodes output '┐'-shaped biphasic current. In this instance, currents flow into the return electrodes (ground electrodes) which should be disposed on a substrate, and stimulate unintended bipolar cells. So, the user recognizes the blur state of the '┐' shape. Accordingly, there is a problem that the user may feel the 1000-pixel sub-type artificial retina has resolution similar to that of the 64-pixel epi-type artificial retina.

In order to reduce blurring of images by the cross-talk phenomenon, Korean Patent No. 10-1838150 proposed a dual mode electrode technique. As illustrated in FIG. 3, while the center stimulation electrode is activated, electrodes around the center stimulation electrode prevent a flow of current by acting as a ground reference. Additionally, the ground reference electrode is activated again, and the activated electrode serves as a ground reference electrode, and such an operation is repeated in sequence.

However, the above method has several disadvantages in that it is difficult for the stimulation current to be equally distributed to the surrounding reference electrodes, and in that unnecessary charges may be inserted due to glitches, clock-feedthrough, charge injection, and the like generated while the stimulation electrodes are switched into the ground reference electrodes and the ground reference electrodes are switched into the stimulation electrodes.

Accordingly, there is a demand for a new sub-type artificial retina device capable of suppressing cross-talk.

### Patent Literature

### Patent Documents

Korea Patent No. 10-1838150

### Disclosure

### Technical Problem

The present disclosure has been made to solve the above-mentioned problems occurring in the prior art, and in an aspect of the present disclosure, an object of the present disclosure is to provide a novel sub-type artificial retina device capable of minimizing cross-talk.

### Technical Solution

To accomplish the above-mentioned objects, according to an aspect of the present invention,
there is provided a sub-type artificial retina device
including:
   a photoelectric conversion unit; and
   a retinal nerve cell stimulation unit,
   wherein the photoelectric conversion unit includes a photodiode array having a plurality of photodiodes for generating current by receiving light in response to external visual information projected onto the retina, and an amplifier for amplifying the current generated by the photodiodes.

The retinal nerve cell stimulation unit includes:
a plurality of stimulation electrodes provided on the photoelectric conversion unit, respectively corresponding to the plurality of photodiodes, and generating action potentials toward the retinal nerve cells in response to the current generated by the photodiodes;
return electrodes receiving current to form a ground on the photoelectric conversion unit, and arranged to surround each of the stimulation electrodes to electrically separate any one stimulation electrode from the other stimulation electrodes; and
a switch capable of connecting the stimulation electrode and the return electrode.

The switch and the retinal nerve cells are connected in parallel between the stimulation electrode and the return electrode, and current flows from the stimulation electrode to the return electrode through the retinal nerve cells in a case in which the switch is open, and flows from the stimulation electrodes to the return electrode without passing through the retinal nerve cells
in a case in which the switch is connected.

In another aspect of the present invention,
there is provided a driving method of the sub-type artificial retina device including
the operations of:
   (S1) generating and amplifying current from a photoelectric conversion unit in response to external visual information projected to the retina;
   (S2) transmitting the current to at least a portion of a plurality of stimulation electrodes;
   (S3) stimulating retinal nerve cells corresponding to the stimulation electrodes to which the current is transmitted; and
   (S4) connecting a switch to remove charge remaining on the retinal nerve cells after the stimulation of the retinal nerve cells is finished.

In a further aspect of the present invention,
there is provided a manufacturing method of the sub-type artificial retina device including
the operations of:
   exposing a top metal layer on top of a photoelectric conversion unit;
   depositing a conductive material on the photoelectric conversion unit; and
   removing the conductive material from an area excluding a portion in which stimulation electrodes and return electrodes will be located.

### Advantageous Effects

The sub-type artificial retina device and the driving method thereof provided in one aspect of the present disclosure can control cross-talk more effectively than the conventional method.

In addition, the method of manufacturing a sub-type artificial retinal device provided in another aspect of the present disclosure can manufacture the sub-type artificial retinal device capable of effectively controlling cross-talk through a simple process and reduce manufacturing costs.

### Description of Drawings

FIG. 1 is a schematic diagram illustrating an installation position according to types of artificial retinas.
FIG. 2 is a schematic diagram illustrating a cross-talk phenomenon of a photodiode array having a conventional return electrode arrangement.
FIG. 3 schematically illustrates a circuit diagram of one among the conventional arts for suppressing cross-talk.
FIG. 4 illustrates a state of an eyeball to which a sub-type artificial retinal device according to an embodiment of the present disclosure is applied.
FIG. 5 is a schematic diagram illustrating a cross section of the sub-type artificial retinal device according to an embodiment of the present disclosure.
FIG. 6 is a schematic diagram illustrating a circuit diagram of the sub-type artificial retinal device according to an embodiment of the present disclosure.
FIG. 7 is a schematic diagram illustrating a manufacturing process of the sub-type artificial retina device according to an embodiment of the present disclosure.
FIG. 8a schematically illustrates a cross-section of a sub-type artificial retina device according to an embodiment of the present disclosure.
FIG. 8b schematically illustrates a cross-section of a sub-type artificial retina device according to a comparative example of the present disclosure.
FIGS. 9 to 12 schematically illustrate COMSOL simulation results when the artificial retinal device according to the embodiment and the artificial retinal device according to the comparative example of the present disclosure are stimulated according to an experimental example of the present disclosure.

### Best Mode

Hereinafter, the present disclosure will be described in detail with reference to the contents described in the accompanying drawings. However, the present disclosure is not restricted or limited by the exemplary embodiments. The same reference numerals in each drawing indicate members performing substantially the same functions.

Objects and effects of the present disclosure can be naturally understood or made clearer by the following description, and the objects and effects of the present disclosure are not limited only by the following description. Additionally, in the description, details of well-known features and techniques may be omitted to avoid unnecessarily obscuring the presented embodiments.

In an aspect of the disclosure,
a sub-type artificial retina device includes:
a photoelectric conversion unit; and
a retinal nerve cell stimulation unit,
wherein the photoelectric conversion unit includes a photodiode array having a plurality of photodiodes for generating current by receiving light in response to external visual information projected onto the retina, and an amplifier for amplifying the current generated by the photodiodes.

The retinal nerve cell stimulation unit includes:
a plurality of stimulation electrodes provided on the photoelectric conversion unit, respectively corresponding to the plurality of photodiodes, and generating action potentials toward the retinal nerve cells in response to the current generated by the photodiodes;
return electrodes receiving current to form a ground on the photoelectric conversion unit, and arranged to surround each of the stimulation electrodes to electrically separate any one stimulation electrode from the other stimulation electrodes; and
a switch capable of connecting the stimulation electrode and the return electrode.

The switch and the retinal nerve cells are connected in parallel between the stimulation electrode and the return electrode.

Current flows from the stimulation electrode to the return electrode through the retinal nerve cells in a case in which the switch is open.

The current flows from the stimulation electrodes to the return electrode without passing through the retinal nerve cells in a case in which the switch is connected.

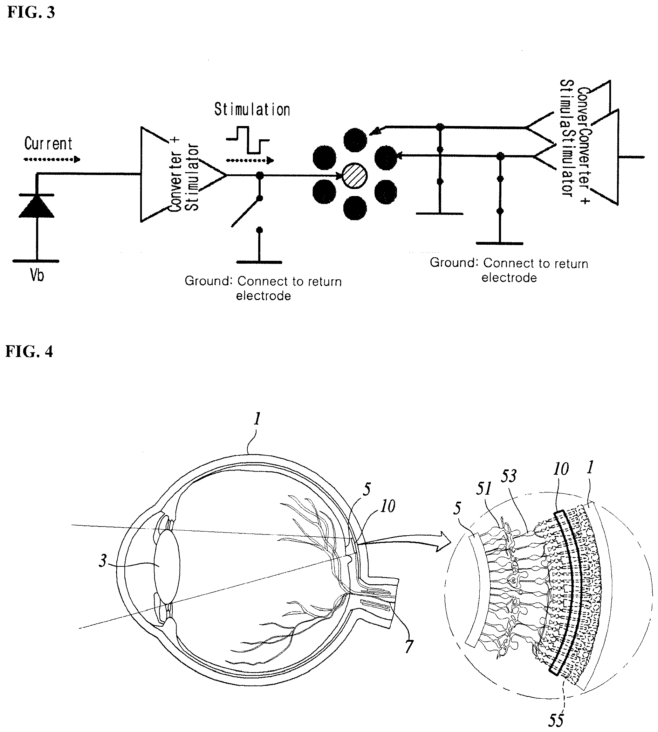

FIG. 4 illustrates a state of an eyeball to which a sub-type artificial retinal device 10 according to an embodiment of the present disclosure is applied. The eyeball includes a retina 5, a nervous tissue 7, a choroid, a sclera, a cornea 1, a pupil 3, an iris, and a ciliary body. As described above, the sub-type artificial retina device 10 is positioned behind the retina 5. The retina 5 has a multilayer structure of retinal nerve cells (ganglion cells), amacrine cells, bipolar cells, horizontal cells, rod cones, and pigment epithelium. For convenience of explanation, in FIG. 4, the retina 5 is largely divided into a retinal nerve cell layer 51, a bipolar cell layer 53, and a rod cone layer 55. The artificial retina device 10 according to the present embodiment may be positioned to replace the rod cone layer 55. That is, the artificial retina device is inserted into a portion in which the rod cone layer 55 is destroyed and disappeared. Since both the retinal nerve cells 51 and the bipolar cells 53 are transparent cell layers, the light incident on the retina 5 is projected to reach the artificial retina device 10. The artificial retina device 10 can use the intermediate signal processing of nerve cells in the internal retinal layer as it is. In this process, the artificial retina device 10 according to the present embodiment provides visual information with high resolution to the user since the stimulation electrodes reacting to external visual information directly stimulate the corresponding bipolar cells 53 but do not stimulate unnecessary bipolar cells 53. Hereinafter, the configuration of the present device will be described as follows.

First, in an aspect of the present disclosure, the sub-type artificial retina device 10 includes a photoelectric conversion unit 100.

The photoelectric conversion unit includes a photodiode array 101 including a plurality of photodiodes receiving light in response to the external visual information projected onto the retina to generate current.

Moreover, the photoelectric conversion unit includes an amplifier 102 amplifying the current generated by the photodiodes.

The photoelectric conversion unit including the stimulator can maximize stimulation efficiency by providing sufficient stimulation even with a small amount of current, and does not need lots of diodes so that the artificial retina device can be miniaturized.

In addition, the photoelectric conversion unit further includes a pulse shaper 103 for converting the generated current into biphasic current.

In an embodiment, the photoelectric conversion unit is connected to the photodiodes, the amplifier, and the pulse shaper in order, but is not limited thereto.

In an embodiment, the photoelectric conversion unit may be a CMOS image sensor.

Additionally, the photoelectric conversion unit includes a top metal layer.

Next, in an aspect of the present disclosure, the sub-type artificial retina device includes a retinal nerve cell stimulation unit 200.

The retinal nerve cell stimulation unit 200 includes a plurality of stimulation electrodes 201 disposed on the photoelectric conversion unit, respectively corresponding to the plurality of photodiodes, and generating action potentials toward the retinal nerve cells in response to the current generated by the photodiodes.

In addition, the retinal nerve cell stimulation unit 200 includes return electrodes receiving current to form a ground on the photoelectric conversion unit, and arranged to surround each of the stimulation electrodes to electrically separate any one stimulation electrode from the other stimulation electrodes.

In this instance, to electrically separate the electrodes from each other means that current does not flow between the electrodes without adding additional parts.

Furthermore, the retinal nerve cell stimulation unit includes a switch 203 capable of connecting the stimulation electrode and the return electrode.

The return electrode comes into contact with the top metal layer. In an embodiment, the return electrode is deposited on the top metal layer along the top metal layer.

The return electrode forms a boundary of a pixel including any one among the plurality of stimulation electrodes.

Each pixel has a polygonal or circular cross-section, but is not limited to such a cross-section having the specific shape. In an embodiment, the pixel has a hexagonal cross-section or a rectangular cross-section.

A distance between any two adjoining stimulation electrodes among the plurality of stimulation electrodes is constant, and the pixels are constant in shape and size.

The average distance between any two adjoining stimulation electrodes among the plurality of stimulation electrodes may be 100 µm or less, preferably 60 µm or less, and more preferably 50 µm or less. The lower the average distance between any two adjoining stimulation electrodes is, the more pixels are formed in the same area.

According to an embodiment of the present disclosure, about 4000 pixels may be applied within 5 mm x 5 mm, which is the size of the fovea as an important size limiting factor of the artificial retina.

In addition, each of the stimulation electrodes is located at the center of each pixel.

The stimulation electrode and the return electrode are made of at least one material selected from the group consisting of platinum, gold, iridium, and iridium oxide. These materials are biocompatible materials and are harmless even if inserted into the body.

The switch and the retinal nerve cells are connected in parallel between the stimulation electrode and the return electrode.

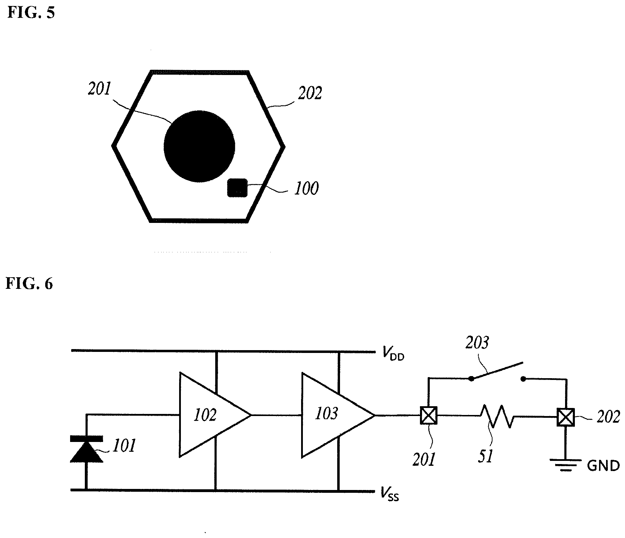

Accordingly, in a case in which the switch is open, the current flows from the stimulation electrode to the return electrode through the retinal nerve cells. In a case in which the switch is connected, the current flows from the stimulation electrodes to the return electrode without passing through the retinal nerve cells (FIG. 6).

When the switch is connected, the charge remaining after the stimulation of the retinal nerve cells can be removed, thereby ensuring biological safety.

In an aspect of the present disclosure, the sub-type artificial retina device can minimize the cross-talk phenomenon by disposing the return electrodes to form a ground reference wall between the stimulation electrodes. Accordingly, a stimulated range is very fine, and as a result, the resolution is remarkably improved.

In another aspect of the present disclosure,
a driving method of the sub-type artificial retina device includes
the operations of:
   (S1) generating and amplifying current from the photoelectric conversion unit in response to the external visual information projected to the retina;
   (S2) transmitting the current to at least a portion of the plurality of stimulation electrodes;
      (S3) stimulating the retinal nerve cells corresponding to the stimulation electrodes to which the current is transmitted; and
      (S4) connecting the switch to remove charge remaining on the retinal nerve cells after the stimulation of the retinal nerve cells is finished.

Hereinafter, in another aspect of the present disclosure, the driving method of the sub-type artificial retina will be described in detail for each operation.

First, the driving method of the sub-type artificial retina device provided in another aspect of the present disclosure includes (S1) generating and amplifying current in the photoelectric conversion unit in response to the external visual information projected to the retina.

In this instance, the photodiodes receive light to generate current, and the amplifier amplifies the generated current.

Moreover, the operation S1 further includes converting the generated current into biphasic current, and the operation is performed by a pulse shaper.

Next, in another aspect of the present disclosure, the driving method of the sub-type artificial retina device includes (S2) transmitting the current to at least a portion of the plurality of stimulation electrodes.

That is, the current is transmitted to the stimulation electrodes corresponding to the photodiodes generating the current in operation S1.

Next, in another aspect of the present disclosure, the driving method of the sub-type artificial retina device includes (S3) stimulating the retinal nerve cells corresponding to the stimulation electrodes to which the current is transmitted.

Accordingly, the optic nerve is stimulated to recognize visual images.

Next, in another aspect of the present disclosure, the driving method of the sub-type artificial retinal device includes (S4) connecting the switch to remove the charge remaining in the retinal nerve cells after the stimulation of the retinal nerve cells is finished.

In a case in which the retinal nerve cells are stimulated through the operation 3, the switch is opened and current flows from the stimulation electrodes to the return electrodes through the retinal nerve cells. However, after the stimulation of the retinal nerve cells in operation S3 is finished, the switch is connected so that the current flows from the stimulation electrodes to the return electrodes without passing the retinal nerve cells, thereby removing the residual charge remaining after the stimulation.

In addition, operations 1 to 4 are repeatedly performed according to external images.

In another aspect of the present disclosure, a manufacturing method of the sub-type artificial retina device can minimize cross-talk between electrodes, maximize stimulation efficiency even with a small-sized sub-type artificial retina device, and remove residual charges to secure biological safety.

In another aspect of the disclosure,
the manufacturing method of the sub-type artificial retina device includes
operations of:
   exposing a top metal layer on top of the photoelectric conversion unit;
   depositing a conductive material on the photoelectric conversion unit; and
   removing the conductive material from an area excluding a portion in which the stimulation electrodes and the return electrodes will be located.

In this instance, in the sub-type artificial retina device, the photoelectric conversion unit includes a top metal layer, and the return electrodes are in contact with the top metal layer.

First, in another aspect of the present disclosure, the manufacturing method of the sub-type artificial retina device includes exposing the top metal layer on the top of the photoelectric conversion unit.

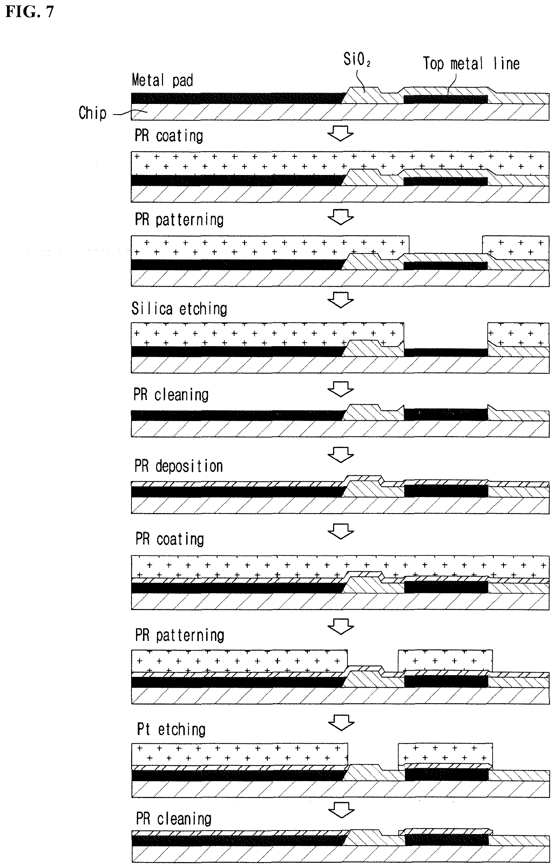

As illustrated at the top of FIG. 7, the top metal layer of the photoelectric conversion unit is covered with SiO₂.

In an embodiment, the above operation is carried out through the operations of coating photoresist (PR) on the photoelectric conversion unit (the second view of FIG. 7), exposing a SiO₂ portion to be etched after irradiating light onto a metal mask on which a pattern is formed (the third view of FIG. 7); etching only the SiO₂ layer (the fourth view of FIG. 7); and removing residual photoresist (the fifth view of FIG. 7), but is not limited to such a method or sequence.

Next, in another aspect of the present disclosure, the manufacturing method of the sub-type artificial retina device includes depositing a conductive material on the photoelectric conversion unit (the sixth view of FIG. 7).

The conductive material is at least one selected from the group consisting of platinum, gold, iridium, and iridium oxide. These materials are biocompatible materials and are harmless even if inserted into the body.

The conductive material may finally constitute the stimulation electrodes and the return electrodes.

In an embodiment, the deposition may be performed by vapor deposition, but is not limited to the specific method.

Next, in another aspect of the present disclosure, the manufacturing method of the sub-type artificial retina device includes removing the conductive material from an area excluding a portion in which the stimulation electrodes and the return electrodes will be located.

In this instance, the portion in which the return electrode is positioned may be a portion coming into contact with the top metal layer, and more specifically, a portion of a conductive material deposited on the top metal layer may function as the return electrode.

In an embodiment, the above operation is carried out through the operations of exposing the conductive material portion to be etched by irradiating light on the metal mask after applying the photoresist (the seventh and eighth views of FIG. 7), removing the exposed conductive material layer (the ninth view of FIG. 7), and removing the remaining photoresist (the tenth view of FIG. 7), but is not limited to such method or sequence.

In another aspect of the present disclosure, the manufacturing method of the sub-type artificial retina device can manufacture a sub-type artificial retina device capable of minimizing cross-talk just by adding a process for the photoelectric conversion unit, does not need complicated design and manufacturing process, and can significantly lower the unit cost of products.

### Mode for Disclosure

Hereinafter, the present disclosure will be described in more detail through embodiments and experimental examples. The scope of the present disclosure is not limited to specific embodiments, and should be construed by the appended claims. In addition, those skilled in the art should understand that many modifications and variations are possible without departing from the scope of the present disclosure.

### <Embodiment 1> Manufacturing of sub-type artificial retina device

A CMOS image sensor chip having the same form as the first view of FIG. 7 was used as the photoelectric conversion unit. In this instance, the CMOS image sensor chip includes a photodiode array and an amplifier, and may further include a pulse shaper.

After the above process is completed, the stimulation electrode is positioned in the metal pad region illustrated in the first view of FIG. 7, and the return electrode is disposed on the top metal layer region after the process is completed.

A photoresist is coated on the CMOS image sensor chip by a spin coating method (the second view of FIG. 7), and a SiO₂ portion to be etched is exposed after light is irradiated onto the metal mask on which a pattern is formed (the third view of FIG. 7), only the SiO₂ layer is etched by a dry etching method (the fourth view of FIG. 7), and residual photoresist is removed by a wet etching method (the fifth view of FIG. 7).

Next, a platinum (Pt) layer is deposited on the entire specimen on the CMOS image sensor chip from which the SiO₂ layer is partially removed (the sixth view in FIG. 7), and photoresist is coated and light is irradiated onto the metal mask to expose the platinum (Pt) portion to be etched (the seventh and eighth views in FIG. 7). Thereafter, the Pt layer exposed by the dry etching method is removed (the ninth view of FIG. 7), and then, the photoresist is removed by wet etching (the tenth view of FIG. 7).

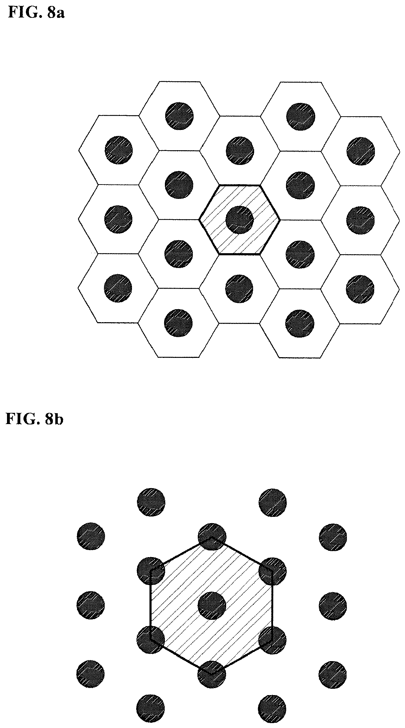

FIGS. 5 and 8a show the cross-section of a structure of the electrode manufactured through the above, and FIG. 6 illustrates a circuit structure of the electrode.

### <Comparative Example 1> Sub-type artificial retina device using dual-mode electrode technique

There is a dual-mode electrode method to prevent a phase blur caused by cross-talk, and the circuit structure of such an example can be confirmed through FIG. 3.

While the center electrode is stimulated, electrodes around the center electrode acts as a ground reference to prevent current flow. In a case in which the ground reference electrode is stimulated again, the previously stimulated electrode serves as a ground reference electrode, and this operation is repeated in order.

FIG. 8b illustrates a cross section of the electrode structure of the comparative example 1, and more detailed contents are described in Korean Patent No. 10-1838150.

### <Experimental Example>

With respect to the electrode structures of Embodiment 1 and Comparative Example 1, in order to confirm intensity of voltage and a stimulation range when electrical stimulation was applied to the stimulation electrode, a COMSOL Physics simulator was used.

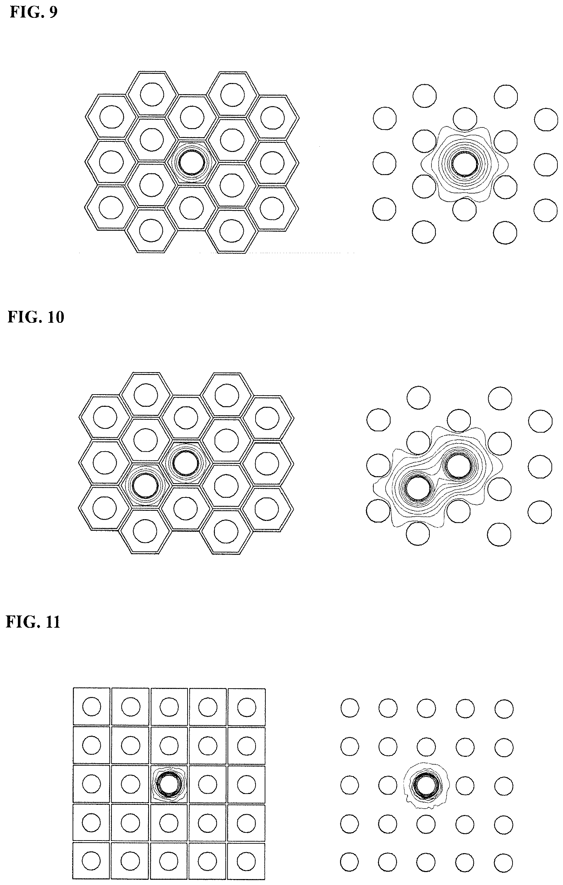

FIGS. 9 to 12 illustrate the results of Embodiment 1 and Comparative Example 1.

In FIGS. 9 to 12, the result of Embodiment 1 is shown in the left side, and the result of Comparative Example 1 is shown in the right side.

In a case in which the pixel has a hexagonal cross section as illustrated in FIGS. 9 and 10, in Embodiment 1, the stimulation range is very narrow due to the return electrodes forming the boundary of each pixel. On the other hand, in Comparative Example 1, since the stimulation range is relatively wide, the possibility of cross-talk between the electrodes is high.

Moreover, in Embodiment 1, through FIG. 10, it is confirmed that stimulations can be discriminated clearly even in a case in which two neighboring electrodes are stimulated simultaneously.

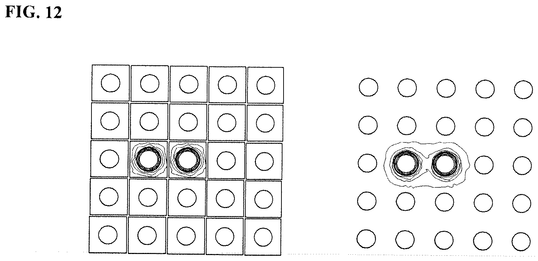

In FIGS. 11 and 12, the pixel has a rectangular cross section. Even in such a shape, the stimulation range is very narrow due to the return electrodes forming the boundary of each pixel. On the other hand, in Comparative Example 1, since the stimulation range is relatively wide, the possibility of cross-talk between the electrodes is high.

Moreover, in Embodiment 1, through FIG. 12, it is confirmed that stimulations can be discriminated clearly even in a case in which two neighboring electrodes are stimulated simultaneously.

That is, it is confirmed that the electrode structure manufactured in Example 1 can effectively control cross-talk regardless of the shape of the pixel, and thus, can be applied to various electrode array structures.

### [Explanation of Reference Numerals]

1: Cornea
3: Pupil
5: Retina
7: Optic nerve
8: Epi-type artificial retina device
9: Sub-type artificial retina device
10: Artificial retina device
51: Retinal nerve cells
53: Bipolar cell
55: Rod cone
100: Photoelectric conversion unit
101: Photodiode (array)
102: Amplifier
103: Pulse shaper
200: Retinal nerve cell stimulation unit
201: Stimulation electrode
202: Return electrode
203: Switch

## Claims

1. A sub-type artificial retina device comprising:
a photoelectric conversion unit; and
a retinal nerve cell stimulation unit,
wherein the photoelectric conversion unit comprises a photodiode array having a plurality of photodiodes for generating current by receiving light in response to external visual information projected onto the retina, and an amplifier for amplifying the current generated by the photodiodes,
wherein the retinal nerve cell stimulation unit comprises:
a plurality of stimulation electrodes provided on the photoelectric conversion unit, respectively corresponding to the plurality of photodiodes, and generating action potentials toward the retinal nerve cells in response to the current generated by the photodiodes;
return electrodes receiving current to form a ground on the photoelectric conversion unit, and arranged to surround each of the stimulation electrodes to electrically separate any one stimulation electrode from the other stimulation electrodes; and
a switch capable of connecting the stimulation electrode and the return electrode, and
wherein the switch and the retinal nerve cells are connected in parallel between the stimulation electrode and the return electrode, and current flows from the stimulation electrode to the return electrode through the retinal nerve cells in a case in which the switch is open, and flows from the stimulation electrodes to the return electrode without passing through the retinal nerve cells in a case in which the switch is connected.

2. The device according to claim 1, wherein the photoelectric conversion unit comprises a top metal layer, and
wherein the return electrode comes into contact with the top metal layer.

3. The device according to claim 1, wherein the return electrode forms a boundary of a pixel including any one among the plurality of stimulation electrodes.

4. The device according to claim 1, wherein the pixel has a polygonal or circular cross-section.

5. The device according to claim 1, wherein the photoelectric conversion unit further comprises a pulse shaper converting the generated current into biphasic current.

6. The device according to claim 1, wherein a distance between any two adjoining stimulation electrodes among the plurality of stimulation electrodes is constant.

7. The device according to claim 3, wherein the pixels are constant in shape and size.

8. The device according to claim 4, wherein each of the stimulation electrodes is located in the center of each pixel.

9. The device according to claim 1, wherein the stimulation electrode and the return electrode are made of at least one material selected from the group consisting of platinum, gold, iridium, and iridium oxide.

10. A driving method of the sub-type artificial retina device of claim 1, comprising the operations of:
(S1) generating and amplifying current from a photoelectric conversion unit in response to external visual information projected to the retina;
(S2) transmitting the current to at least a portion of a plurality of stimulation electrodes;
(S3) stimulating retinal nerve cells corresponding to the stimulation electrodes to which the current is transmitted; and
(S4) connecting a switch to remove charge remaining on the retinal nerve cells after the stimulation of the retinal nerve cells is finished.

11. The driving method according to claim 10, wherein the operations 1 to 4 are performed repeatedly.

12. A manufacturing method of the sub-type artificial retina device of claim 2, comprising the operations of:
exposing a top metal layer on top of a photoelectric conversion unit;
depositing a conductive material on the photoelectric conversion unit; and
removing the conductive material from an area excluding a portion in which stimulation electrodes and return electrodes will be located.

13. The manufacturing method according to claim 12, wherein the conductive material is at least one selected from the group consisting of platinum, gold, iridium, and iridium oxide.
